# EUROPEAN PATENT SPECIFICATION

(11) **EP 2 729 204 B1**
(45) Date of publication and mention of the grant of the patent: **20.11.2024**
(21) Application number: 12806948.1
(22) Date of filing: 04.07.2012
(51) Int. Cl.: A61M 5/32

(54) **NEEDLE GUARD WITH AN ACTIVE STATE AND A PASSIVE STATE**
NADELSCHUTZ MIT EINEM AKTIVSTATUS UND EINEM PASSIVSTATUS
PROTECTION D'AIGUILLE AYANT UN ÉTAT ACTIF ET UN ÉTAT PASSIF

(30) Priority: 05.07.2011 SE 1150633; 05.07.2011 US 201161504560 P; 20.12.2011 SE 1151224
(43) Date of publication of application: 14.05.2014
(73) Proprietor: Greiner Bio-One GmbH, 4550 Kremsmünster (AT)
(72) Inventor: DOMONKOS, Robert, S-234 36 Lomma (SE)
(74) Representative: Burger, Hannes
(86) International application number: PCT/SE2012/050771
(87) International publication number: WO 2013/006134

(56) References cited:
- WO-A1-2008/076459
- DE-A1- 102008 002 796
- US-A- 5 132 369
- US-A- 5 242 417
- US-A1- 2006 270 979
- US-A1- 2008 208 138
- US-A1- 2008 269 693

## Description

### TECHNICAL FIELD

The present invention relates to a needle guard arranged to shield a needle from accidental contact.

### BACKGROUND

The clinical utilization of a pointed hollow needle is well known in the medical art for the administration of solutions or suspensions, such as medicaments, to a human or animal. After puncturing of the skin and introduction of the needle tip, the content of a syringe, typically connected to the rear part of the needle, is administered to the human or animal through the hollow needle. The needle has then done its duty and is withdrawn. An unprotected withdrawn needle constitutes, however, a serious health hazard due to the fact that it may be contaminated with e.g. infectious agents originating from the patient's blood or other body fluids, in combination with the needle tip's inherent ability to easily penetrate skin. Hence, the medical personnel who are handling the withdrawn needle may acquire the corresponding disease, e.g. HIV or hepatitis, if by accident contacting it with their skin. In order to circumvent or alleviate the health hazards associated with such a withdrawn needle amongst other things, there has been much effort devoted to the development of various kinds of needle tip protectors.

US5603699 discloses a needle guard assembly comprising a mounting base and a jacket assembly pivotally mounted on the mounting base. The jacket assembly comprises a shield member, a lever member and activating means. The activating means are adapted for movement of the j acket assembly from a retracted position to a closed position around a needle. Disadvantages of this needle guard assembly include its complicated construction and inherent bulkiness. The former being associated with a large cost of production and the latter with at least an increased volume of bio-hazard waste. Furthermore, a multi-part needle guard such as this is associated with a relatively high cost of production and difficulties in its assembly.

EP0887082 A2 describes a safety cover which is pivoting around a point near the needle hub. The safety cover is folded forwards and to the side over the needle after injection and withdrawal of the needle from the skin. To assure sufficient locking force, the pivoting safety cover is provided with a locking hook mechanism that mates with the needle hub upon locking, which in turn demands the manual push of the pivoting safety cover to be sufficient to overcome the resistance of the locking hook mechanism.

This force needs to be relatively high, which increases the risk of the user not to lock the safety cover in e.g. a stressful situation. The safety cover may thus be left unlocked which results in a potential health hazard. Also, the needle tip is not secured until the pivoting arm has been manually pushed against the needle shaft, before which the user still is susceptible to stung/cut.

Additional safety covers of the prior art with a construction similar to the construction of the safety cover described in EP0887082 A2, such as the safety cover disclosed in US4982842, may comprise an additional looking hook mechanism that locks around the shaft of the needle, or only a looking hook mechanism that locks around the shaft of the needle. The force needed to overcome the resistance of the locking hook mechanism needs, also in these cases, to be quite high. This force is also applied on the needle. The needle may therefore be broken or lost from the cooperation with the hub. This presents a danger to the user, since the user may be stung or cut on the broken/lost needle.

Document WO 1993/016745 and WO 2009/007718 discloses a further needle guard for protecting a needle.

None of above described needle guards protects the needle immediately after withdrawal of the needle from the skin. Hence, a user may accidently come in contact with the needle tip in the time span between the withdrawal of the needle and the activation of the needle guard by the user. It is well known that the patient often moves during the painful administration of e.g. medicaments through the needle, i.e. when the content of the connected syringe is injected. Such a movement by the patient may thus result in involuntary withdrawal of the needle, leaving this exposed and without protection from accidental contact. It is estimated that 20 to 25% of all accidents, in which medical personnel gets injured by the needle during injection, is occurring during the actual injection of the content of the syringe.

Despite general knowledge of the problem of accidents that occur during and due to injection of medicaments for the past 10 to 20 years, no automatic shielding device of inj ection needles that addresses and solves this problem has yet been described.

Hence, an improved needle guard for automatic shielding of a needle during and after its employment for e.g. injection is desired.

### SUMMARY

It is an object of the present invention, considering the disadvantages mentioned above, to provide an improved needle guard which automatically protects the needle immediately after withdrawal of the needle from the skin of a patient.

It is another object of the present invention, to provide a needle guard which may be mounted on a wide range of standard needles or on the assembly consisting of a standard needle and standard syringe.

It is another object of the present invention, to provide a needle guard which may be produced in one piece.

These and other objects, which will appear from the following description, have now been achieved by a needle guard for protecting a needle arranged in connection to the needle guard from accidental contact, comprising: a spring loaded needle shield being pivotable from an open position in which the needle shield is separated from the needle to a closed position in which the needle shield is enclosing and protecting the needle; and a mounting base having a constant spatial relationship with the needle when the needle guard is being used, the mounting base being movably connected to the needle shield for enabling movement between the open and the closed position; wherein the needle shield is having a passive state and an active state, the needle shield being urged towards the closed position when in the active state.

According to another aspect of the present invention, the needle guard may comprise a hinge structure connecting the needle shield to the mounting base for forcing the needle shield into one of either said open position or said closed position; wherein the hinge structure has a dead-center position, such that the needle shield is in the passive state whereby it is being forced by the hinge structure from a first position into the open position if the dead-center position has to be passed to reach the closed position, and in the active state whereby it is being forced by the hinge structure from a second position into the closed position if the dead-center position has to be passed to reach the open position.

According to yet another aspect of the present invention, the hinge structure may comprise at least one toggle joint and at least one tension member. The at least one toggle joint and the at least one tension member may be connected to the needle shield and to the mounting base.

According to yet another aspect of the present invention, the mounting base may comprise a needle hub bore for attachment to a needle hub, from which needle hub the needle is extending.

According to yet another aspect of the present invention, the mounting base may be integrated with the needle hub, from which needle hub the needle is extending, such that the mounting base and the needle hub are parts of the same monolithically formed article. When the mounting base is integrated with the needle hub, the mounting base is preferably provided with connection means, such as e.g. a female fitting, for connection to the distal end of a syringe. Such connection means are typically a part of the needle hub.

According to yet another aspect, the mounting base may comprise means for connection to both a needle hub, from which the needle is extending, and a syringe. According to this aspect, the mounting base, carrying the needle guard, is positioned intermediately of the needle hub and the syringe. The mounting base is then arranged distally of the syringe and proximally of the needle hub. The means for connection may comprise at least one of: a male fitting, for connection to the needle hub; and a female fitting, for connection to the syringe. In this way, a needle guard may be provided at standardized needle hubs and syringes, with standardized fittings, such as Luer-Lok^{®} or Luer-Slip^{®}.

According to yet another aspect of the present invention, the dead-center position may be a position in which the angle between the longitudinal extension of the needle and the longitudinal extension of the needle shield is in the range of 45 to 170°, when the mounting base is attached to or integrated with the needle hub from which the needle is extending.

According to yet another aspect, the needle guard may further comprise at least one locking member extending from or forming a part of the needle shield, wherein the at least one locking member may lockingly engage with the mounting base or any extension or recess thereof when the needle shield is in the closed position, to lock the needle shield in the closed position.

According to yet another aspect, the locking member may comprise at least one slit, for allowing the needle shield to lockingly engage with the mounting base by utilization of a relatively low force.

According to yet another aspect, the needle guard may further comprise at least one unidirectionally gated bar extending from the needle shield in the volume enclosed by the needle shield. The unidirectionally gated bar may lockingly engage with the needle when the needle shield is in the closed position, to lock the needle shield in the closed position.

According to yet another aspect, the unidirectionally gated bar may comprise at least one slit, for allowing the needle shield to lockingly engage with the needle by utilization of a relatively low force.

According to yet another aspect, the force needed for the needle shield to lockingly engage with the mounting base or the needle may be less that the force by which the needle shield is being urged towards the closed position when in said active state, such as the force applied by the hinge structure, when forcing the needle shield into the closed position, for allowing locking of the needle shield in the closed position by the force by which the needle shield is being urged towards the closed position when in said active state, such as the force provided by the hinge structure.

According to yet another aspect, the needle guard may be monolithically formed as a single article when manufactured.

According to yet another aspect, the needle guard may be made of a thermoplastic, plastic or polymeric material.

According to yet another aspect, the needle guard may be manufactured by a method comprising the step of molding or injection molding a thermoplastic, plastic or polymeric material.

According to yet another aspect, a needle guard assembly is provided comprising the needle guard, a needle hub and a needle, wherein the needle guard is mounted on or integrated with the needle hub.

According to yet another aspect, a method for the manufacturing of a needle guard assembly is provided, comprising the step of press-fitting or gluing the mounting base on the needle hub when the mounting base is provided with a needle hub bore; press-fitting or gluing the needle in the needle hub when the mounting base is integrated with the needle hub; or connecting the mounting base to the needle hub when the mounting base comprises means for connection to a needle hub.

Further features of the invention and its embodiments are set forth in the appended claims.

### BRIEF DESCRIPTION OF THE DRAWINGS

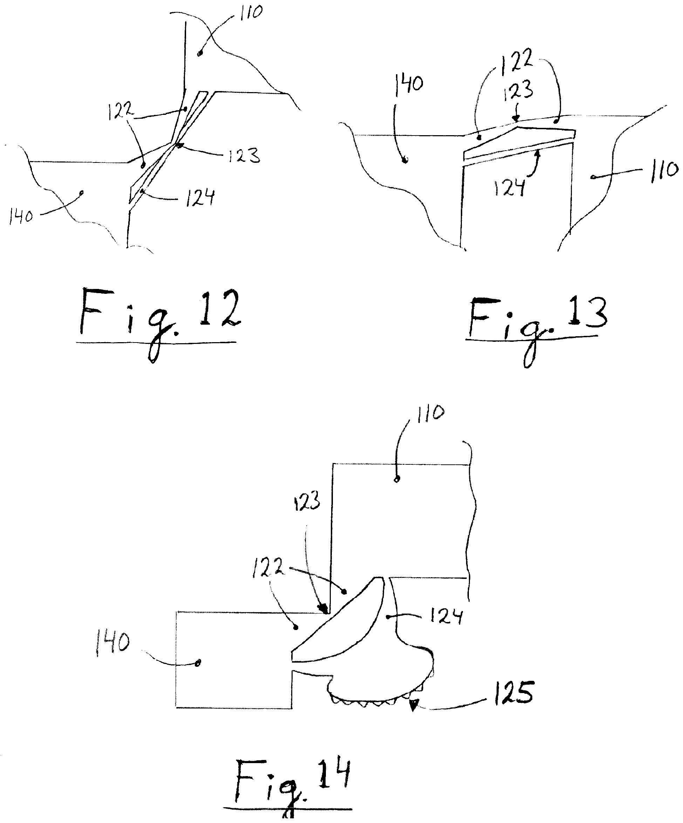

These and other aspects, features and advantages of which the invention is capable will be apparent and elucidated from the following description of non-limiting embodiments of the present invention, reference being made to the accompanying drawings, in which
Fig. 1 is a view from the side of a needle guard in the closed position mounted by means of a mounting base on a needle hub, with a needle being locked by a unidirectionally gated bar and extending from the distal end of the needle hub into the inside of a needle shield and with a syringe mounted in the proximal end of the needle hub, according to one embodiment of the invention;
Fig. 2 is the needle guard of Fig. 1 in the open position, according to one embodiment;
Fig. 3 is a perspective view of a needle guard in the closed position, displaying an elongated slot in the needle shield, in which needle shield the needle is enclosed with exception for the most proximal part, according to one embodiment of the invention;
Fig. 4 is a view from the side of the elongated slot of a needle guard in the closed position with two locking members clamping around the mounting base, in which mounting base is provided with planar surfaces on which the locking members may slide when moving into the locked position, according to one embodiment of the invention;
Fig. 5 is a view from the side of a planar surface of the needle guard of Fig. 4, according to one embodiment of the invention;
Fig. 6 is an expansion view of the needle guard of Fig.4, showing an expansion of the locking member as it clamps around the mounting base, according to one embodiment of the invention;
Fig. 7 is the needle guard of Fig. 6 in a position between a dead-center position and the closed position, showing the distal hook-like end of the locking member being in contact with the planar surface of the mounting base whereby the slit is expanded somewhat in comparison to the same slit of the needle guard of Fig. 4 and 6, according to one embodiment of the invention.
Figs. 8A, 8B, 8C and 8D, are cross sectional views of needle shields in the closed position with a needle enclosed in the volume of each needle shield illustrating various embodiments of elongated slots;
Fig. 9 is a cross sectional view of a needle shield with one unidirectionaly gated bar which is extending inwards into the volume of the needle shield and provided with one slit close to the attachment point, according to one embodiment of the invention;
Fig. 10 is a cross sectional view of a needle shield with two unidirectionaly gated bars extending inwards into the volume of the needle shield and towards each other, each bar being provided with one slit close to the attachment point, according to one embodiment of the invention;
Fig. 11 is a side-ways expansion view of a hinge structure comprising one toggle joint, which toggle joint is comprising two leg portions connected at a toggle joint knee, and one tension member of a needle guard in the closed position, according to one embodiment of the invention;
Fig. 12 is displaying the hinge structure of the needle guard of Fig. 11 in the dead-center position, according to one embodiment of the invention;
Fig. 13 is displaying the hinge structure of the needle guard of Fig. 11 and 12 in the open position, according to one embodiment of the invention;
. Fig. 14 is a side-ways expansion view of a hinge structure, comprising one toggle joint, which toggle joint is comprising two leg portions connected at a toggle joint knee, and a V-shaped tension member with a press member at the point of the V in the distal end of the V-shaped tension member, of a needle guard in the open position, according to one embodiment of the invention;

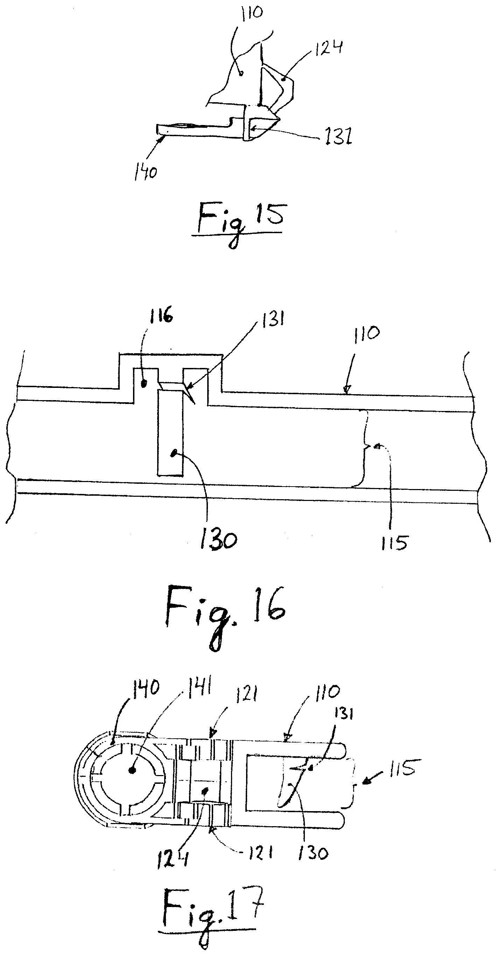

Fig 15 is a view from the side of a mounting base, hinge structure and a part of a needle shield provided with a locking member that clamps around the mounting base in the closed position, according to one embodiment;
Fig. 16 is an expansion view from the side of the elongated slot of a needle shield provided with a slit protecting slot in which the slit of a unidirectionally gated bar resides whereby the slot can not be brought in contact with the needle, according to one embodiment;
Fig. 17 is a view from the top of a needle guard in the open position showing a needle hub bore provided with four slots to mate with corresponding protrusions of a needle hub when mounted therein, according to one embodiment;

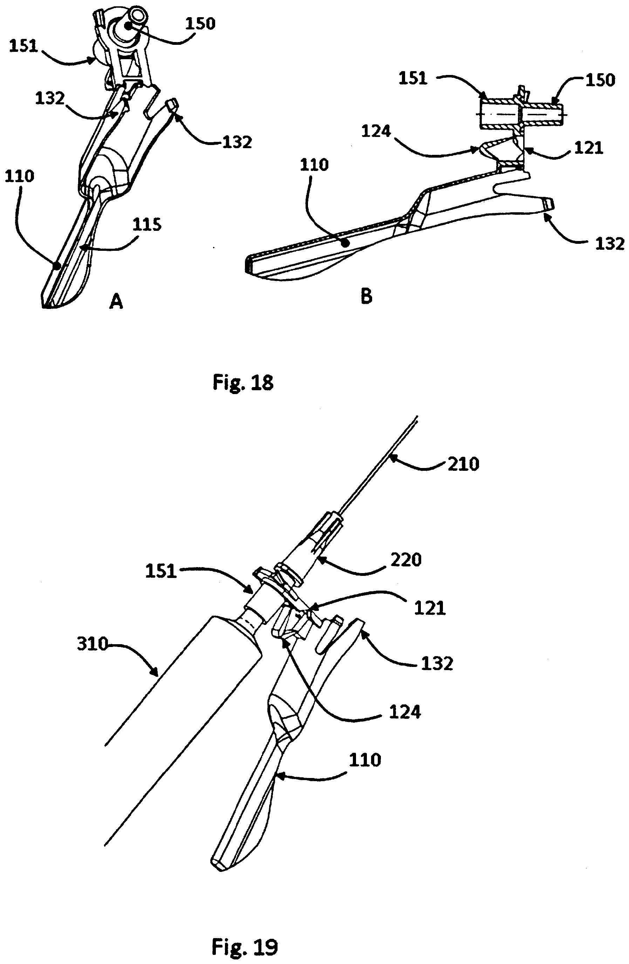

Fig. 18A is a perspective view of a needle guard in the open position without syringe, needle hub and needle, displaying a male fitting onto which a needle hub may be mounted and an oppositely positioned female fitting into which a syringe may be mounted, according to one embodiment of the invention;
Fig 18B is a cross sectional view of the needle guard of Fig. 18A;
Fig 19 is a perspective view of the needle guard of Fig. 18A and 18B, onto which a syringe and a needle hub with a needle has been mounted by pressing the corresponding fitting of the syringe, and the corresponding fitting of the needle hub, into the female fitting and onto the male fitting, respectively, according to one embodiment;

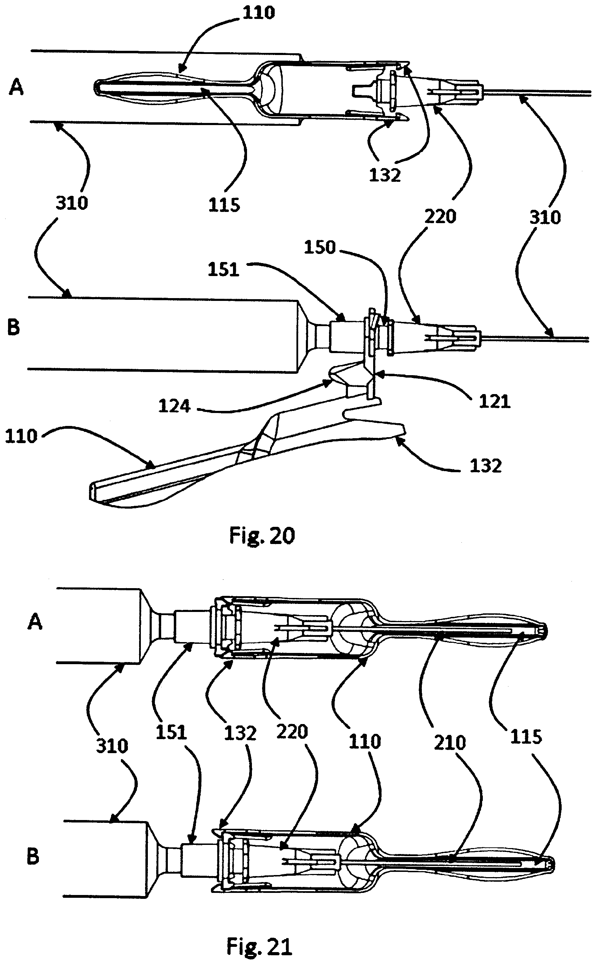

Fig 20A and 20B are views from the side of the needle guard of Fig. 19;
Fig. 21A is the needle guard of Fig. 19, in a position between the dead-center position and the closed position, showing the locking members contacting their counterparts of the mounting base, but not yet locking with these; and
Fig. 21B is the needle guard of Fig. 19, in a closed position, showing the locking members lockingly engaging with their counterparts of the mounting base;

### DETAILED DESCRIPTION OF THE EMBODIMENTS

Embodiments of the present invention will be described in more detail below with reference to the accompanying drawings in order for those skilled in the art to be able to carry out the invention. The invention may, however, be embodied in many different forms and should not be construed as limited to the embodiments set forth herein. Rather, these embodiments are provided so that this disclosure will be thorough and complete, and will fully convey the scope of the invention to those skilled in the art. The embodiments do not limit the invention, but the invention is only limited by the appended patent claims. Furthermore, the terminology used in the detailed description of the particular embodiments illustrated in the accompanying drawings is not intended to be limiting of the invention.

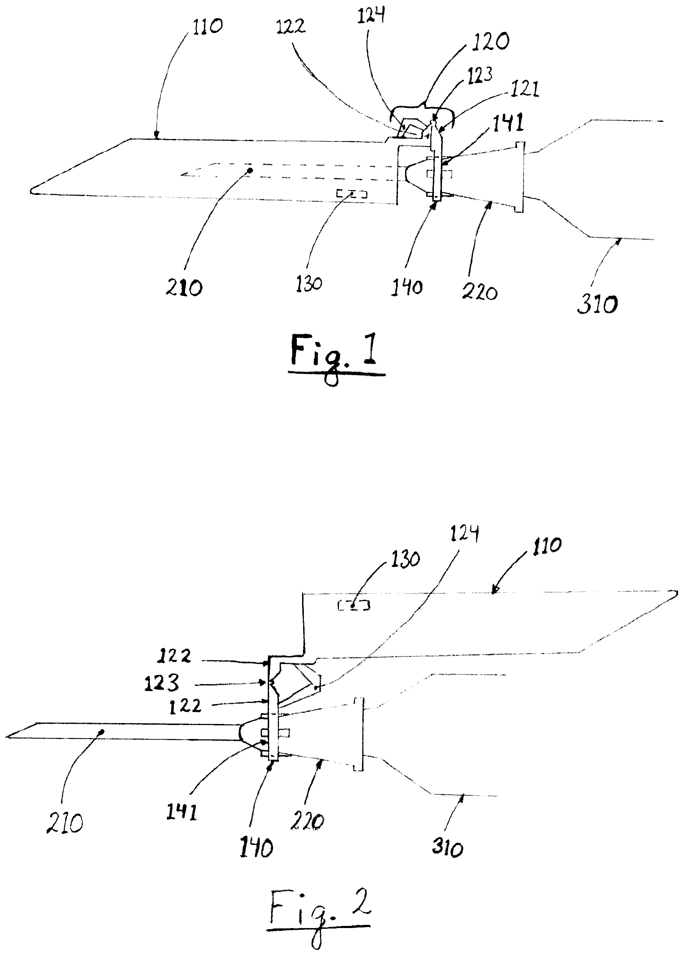

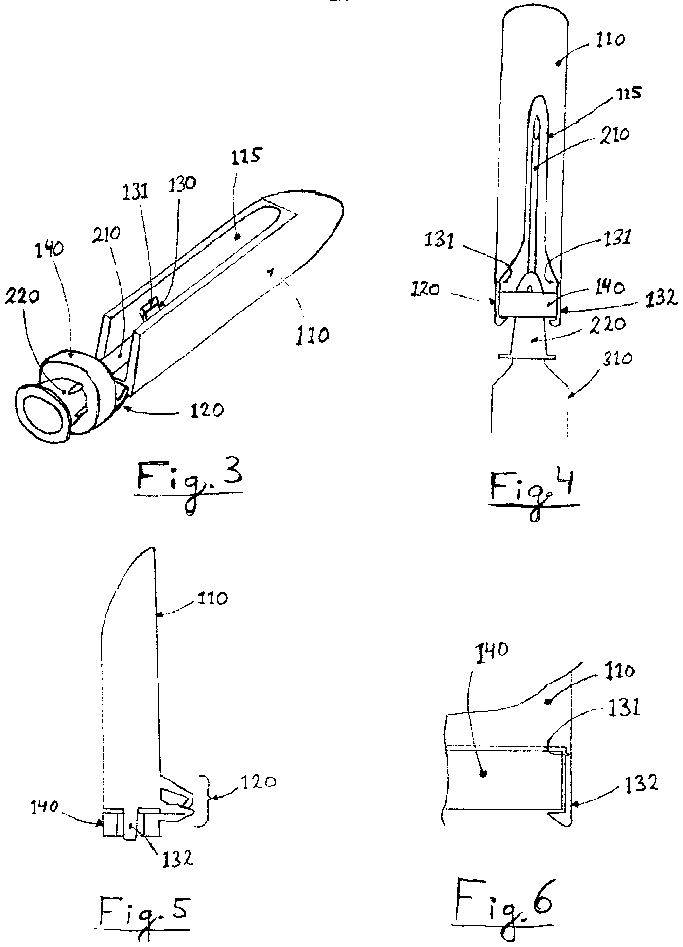

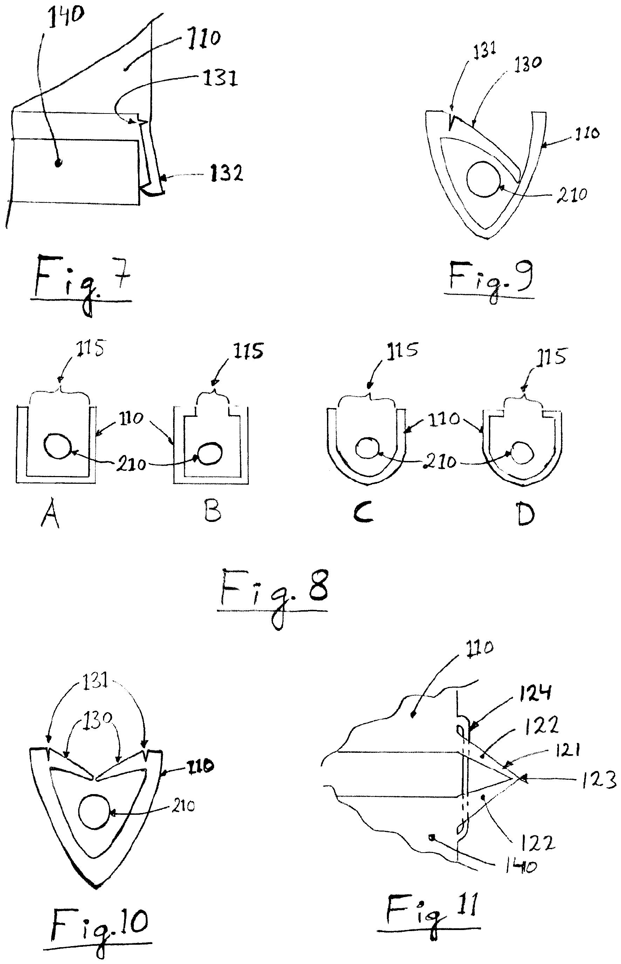

Embodiments of the present invention will now be described below with reference to Figs. 1 to 21. Reference to various parts of the drawings are done by numbers according to the table below.

| number | part |
|---|---|
| 100 | needle guard |
| 110 | needle shield |
| 115 | elongated slot |
| 116 | slit protecting slot |
| 120 | hinge structure |
| 121 | toggle joint |
| 122 | leg portion |
| 123 | toggle joint knee |
| 124 | tension member |
| 125 | press member |
| 130 | unidirectionally gated bar |
| 131 | slit |
| 132 | locking member |
| 140 | mounting base |
| 141 | needle hub bore |
| 150 | male fitting |
| 151 | female fitting |
| 210 | needle |
| 220 | needle hub |
| 310 | syringe |

The needle guard 100 essentially comprises a unique, novel, inventive and advantageous combination of a spring loaded needle shield 110, a mounting base 140 and means for connecting the needle shield 110 to the mounting base 140. The mounting base 140 comprises a lumen with a central axis, such that a liquid may be passed from a proximal end of the mounting base 140 to the distal end thereof, and vice versa. The needle shield 110, which may be shaped as a rod, arm or have any other elongated shape, is pivotably movable in relation to the mounting base 140 from an open position to a closed position. The mounting base 140 is not movable in relationship to a needle 210, arranged on the mounting base 140 or connected via a fitting to the mounting base 140, such that the needle 210 is extending distally from the mounting base 140 and ending distally in the tip of the needle 210, when the needle guard is used, i.e. it is having a constant spatial relationship with the needle 210. The needle 210 is an injection needle, comprising a lumen for delivering a fluid there through. The lumen of the injection needle 210 is in fluid communication with the lumen of the mounting base 140, such that a liquid may be passed from for example a syringe connected to the proximal end of the mounting base 140, through the mounting base 140 and the needle 210, to be expelled at the distal end of the needle 210, i.e. the needle tip of the needle 210. The needle shield 110 is connected to the mounting base 140 by movable means for connection to the mounting base 140 at a pivot point around which the needle shield 110 may pivot. The needle shield 110 may be connected to the mounting base 140 in the vicinity of the proximal end of the needle 210 arranged or connected on/to the mounting base 140. Such movable means for connection may be a hinge structure 120 or other suitable movable means . The movable means for connection may further comprise or be closely located to a resilient part, such as a spring or a tension member 124. When a safety mechanism of the needle guard 100 is activated by a user, i.e. when the needle shield 110 is brought to an active state, the force applied by the resilient part urges the needle shield 110 to a closed position in which the needle shield 110 is enclosing and protecting the needle 210. In the corresponding passive state, the resilient part may or may not urge the needle shield 110 to an open position in which the needle shield 110 is separated from the needle 210. In the open position, and typically also in the passive state, the needle 210 may be used for e.g. injection of medicaments.

The movement of the needle shield 110 may typically occur via a dead-center position of the hinge structure 120. The user's activation of the safety mechanism, i.e. transition from passive to active state, may thus be achieved by movement of needle shield 110 towards closed position just past the dead-center position. The mounting base 140 is configured for being connected to, optionally via e.g. a male fitting 150, or integrated with the needle hub 220 of, typically, a standard needle hub-needle assembly. When the mounting base 140 is provided with a male fitting 150, it is preferably also provided with a corresponding and oppositely directed female fitting 151, for connection to the distal outlet part of a syringe. The content of the syringe may thereby be transferred from the syringe, via a common bore or hole through the female fitting 151 and the male fitting 150, into the needle hub-needle assembly. The resulting needle guard assembly may advantageously be used in various applications, for example, medical applications or applications wherein toxic compositions are transferred via syringes connected to the needle 210. In the open position, the needle shield 110 is remotely positioned in relation to the needle 210 so that the latter may be used for, for example, withdrawing liquid medicaments into a syringe connected to the needle 210 and injecting these into a patient. In the closed position, the needle shield 110 is surrounding or enclosing the needle 210 to such an extent that it can not be accidently touched by a user. A user may manually activate the safety function of the needle guard 100 by moving, directly or indirectly via additionally thereto connected parts, the needle shield 110 from the open position towards the closed position and just past the dead-center position. The needle shield 110 then enters the closed position, or is in an active state from which it will automatically enter the closed position and thereby protect the needle 210 from accidental contact when not hindered by any foreign object, such as the outer surface of a patient. Preferably, the needle guard 100 also comprises a locking mechanism, which locks the needle shield 110 in the closed position so that it may not, or only with great difficulty, be returned to the open position. The force applied by the hinge structure 120 may, in collaboration with the locking mechanism of the needle guard 100, thus lock the needle shield 110 in the closed position without the need of any applied external force. In embodiments where the needle guard 100 is provided with a locking mechanism, the hinge structure 120 is preferably arranged such that it forces the needle shield 110 into the closed position with a force greater than the force needed for the locking mechanism of the needle guard 100 to lock the needle shield 110 in the closed position. The locking mechanism of the needle guard 100 may comprise one or several locking members 132 or one or several unidirectionally gated bars 130, or any other suitable locking mechanism known in the art The locking mechanism of the needle guard 100 may also comprise one or several locking members 132 in combination with one or several unidirectionally gated bars 130, without or in combination with any other suitable locking mechanism known in the art. A user may typically use a needle guard assembly comprising the needle guard 100 in accordance with a three step procedure, as exemplified for the following medical application: (i) the needle shield 110 is in open position when the user penetrates the skin of a patient with the needle 210, (ii) the user activates the safety function by manually moving the needle shield 110 passed the dead-center position whereby the needle shield 110 is set in an active state and may contact the skin of the patient, (iii) the user injects a medicament through the needle 210, and (iv) the user withdraws the needle 210 from the patient whereby the needle shield 110 automatically enters the closed position to protect the needle 210.

The needle guard 100 of the invention automatically, without the need of any additional act by the user, protects the needle 210 immediately after withdrawal of the needle from the skin of a patient. This is a highly advantageous feature of the needle guard 100 of the invention as the needle 210, if accidently released from the skin of the patient during step iii, would be left exposed and thus possess a great health hazard. Similar needle protection systems of the prior art are not automatic in this meaning and do require a manual activation by the user after step iii.

### The needle shield 110

The needle shield 110 may be an elongated structure of various forms, such as a needle shielding arm, adapted, as known in the art, for fully or partly enclosing the needle 210 in its inner volume. At least the tip and the immediately adjacent part of the needle 210 may thereby not be possible to reach, either by accident or voluntarily. The needle shield 110 may be adapted for allowing a sideways entry of the needle 210. For example, by being provided with an elongated slot 115 formed longitudinally along the length thereof. When the needle shield 110 is moved from the open to the closed position, the needle 210 is passing through the slot 115 to be essentially enclosed by the inner volume of the needle shield 110. The width of the elongated slot 115 may be such that facile entry of the needle 210 can occur. For example the ratio between the width of the slot 115 and the diameter of the needle may be 1 to 10, such as 1.1 to 5, or preferably 1.5 to 3. When in the closed position, the lower part of the needle shield 110 may be in contact with the needle hub 220 or the mounting base 220, such that the needle 210 in its entire length is enclosed by the needle shield 110. The lower part of the needle 210, i.e. the part of the needle 210 which is closer to the needle hub 220, may or may not be enclosed by the needle shield 110 when in closed position. Preferably, at least the tip of the needle 210 and 80% of the length of its shaft may be enclosed by the needle shield 110 when in closed position. The needle shield 110 may comprise one or several locking mechanisms, as further explained herein or as well known in the art, for accomplishment of a secure engagement with e.g. the shaft of the needle 210, the needle hub 220 or the mounting base 140, when in the closed position. The needle shield 110 may thus not be returned to the open position, which is beneficial for the safety of the needle guard 100. The needle shield 110 may also be provided with additional elements for improvement of e.g. the safety, appearance or handling of the needle guard 100. Such elements include, for example, integrated ribs for improvement of the capability to resist external forces and coloration for indication of e.g. size of the needle 210. Additional elements include, for example, an absorbing pad for the absorption of blood residues or toxic chemicals after use and a smooth and/or suitable shaping of the external surface that may contact the skin of a patient after activation of the safety mechanism.

According to one embodiment, the back-side of the needle shield 110, i.e. the side opposite the side of the elongated slot 115, may be provide with one or several elements that facilitates the manual movement towards a closed position. For example, the needle shield 110 may be provided with an extension reachable by the finger of a user. The surface may, partly or fully, be roughed or provided with ribs or the like which may increase the friction between the surface and the top of a finger.

According to one embodiment, the needle shield 110 may be provided with one or several slit protecting slots 116 for placement of one or several unidirectionally gated bars 130 therein. Such a slot has an inner volume that may extend essentially perpendicular to the extension of the needle shield 110. The one or several slits 131 of the one or several unidirectionally gated bars 130 may be located in the volume of the slit protecting slot 116. The slits 131 may preferably be located outside the plane of the path of the needle 210 as it is moved from open to closed position. Advantages of one or several slit protecting slots 116 include, for example, the minimized or eliminated risk of the needle 210 getting stuck on a slit 131.

According to one embodiment, the front-side of the needle shield 110, i.e. the same side as the side of the elongated slot 115, may be provide with one or several elements that facilitates the movement on skin. It may, for example, be curved such that there are no edges that contact the skin upon movement to the closed position, such as when withdrawing the needle 210 after injection. Its surface is preferably adapted such that the friction between the same and skin is low. It may, for example, be well polished or made of a low-friction material. Advantages include a minimized risk of malfunction of the safety mechanism of the needle guard 100.

### The mounting base 140

The mounting base 140 may comprise means for securely fitting to and mating with a needle hub 220. Such means may be constituted by a needle hub bore 141 extending through the mounting base 140. The inner surface of the needle hub bore 141 may have essentially the same shape as the outer surface of the needle hub 220 at a section thereof onto which the mounting base 140 is fitted. The spatial extension, such as the diameter, of the needle hub bore 141 may be slightly less than the corresponding spatial extension of the needle hub 220 at a section thereof onto which the mounting base 140 is fitted. When fitted on the needle hub 220, by e.g. press fitting, the mounting base 140 is thereby securely fitted thereon and may not be moved relative the needle hub 220. The optimal difference in spatial extension of the needle hub 220 and the needle hub bore 141 for secure press fitting is well known to the one skilled in the art.

According to one embodiment, the needle hub bore 141 may be a conically shaped hole with a diameter that narrows in the same direction as the extension of the needle 210.

According to one embodiment, the needle hub bore 141 may be provided with longitudinal recesses that correspond and mate with the longitudinal protrusions of standard needle hubs 220. The needle hub bore 141 may be provided with, for example, 2 to 5, such as 3 to 4, or 4 such recesses.

According to one embodiment, the mounting base 140 may be securely fitted to the needle hub 220 by press-fitting the mounting base 140 on the needle hub 220. The needle hub 220 is thereby securely fitted in, for example, the needle hub bore 141 of the mounting base 140. Advantages of a press-fitted mounting base 140 include the lower cost of production in comparison to other means of connection.

According to one embodiment, the mounting base 140 may be securely fitted to the needle hub 220 by gluing. The needle hub 220 may, for example, be glued to the needle hub bore 141 of the mounting base 140.

According to one embodiment, the mounting base 140 may be securely fitted to the needle hub 220 by welding.

According to one embodiment, the mounting base 140 may be integrated with the needle hub 220. The mounting base 140 and the needle hub 220 are thus parts of the same monolithically formed article. The needle 210 may be press-fitted or glued to the needle hub part of this integrated mounting base 140 and needle hub 220 article.

The mounting base 140 may comprise means for fitting to the proximal end of a needle hub-needle assembly and/or to the distal end of a syringe 310. Such means may typically be the type of fittings which allow secure fitting to standard fittings of syringes 310 and needle hubs 220. Examples of such standard fittings include Luer-Lok^{®}, Luer-Slip^{®}, and various types of bayonet sockets or the like, as well known in the art. Preferably, such fittings, e.g. male fittings 150 or female fittings 151, are air tight so that no gas or liquid, such as blood or any other body liquid, may pass between the contact area of these and the corresponding fittings of the needle hub 220 and the syringe 310, respectively. In this way the mounting base 140 will be positioned intermediately of the needle hub-needle assembly and the syringe 310, such that the mounting base 140 is provided distally of the syringe tip, and connected thereto by a fitting arrangement, such as a Luer-Lok^{®} or Luer-Slip^{®} fitting, and proximally of the needle hub-needle assembly, and connected thereto by a fitting arrangement, such as a Luer-Lok^{®} or Luer-Slip^{®} fitting. Essentially, a passage such as e.g. a hole or bore, is enabling fluid passage through the fitting of the mounting base 140 which is connected to the distal end of a syringe 310, such as e.g. a female fitting 151, and further through the fitting of the mounting base 140 which is connected to the needle hub 220, such as e.g. a male fitting 150, to thereby enter the needle hub-needle assembly. Fluid passage from the inside of the syringe 310 to the inside of the needle 210 is thus achieved. The means for fitting to the proximal end of a needle hub- needle assembly and/or to the distal end of a syringe 310 may typically be arranged such that the longitudinal extension of the syringe 310 coincides with the longitudinal extension of the needle 210. A needle guard 100, with a mounting base 140 that comprise means for fitting to a needle hub-needle assembly and a syringe, is highly versatile. For example, large supplies of standard syringes and needle hub-needle assemblies, which currently lack any kind of needle tip protector and therefore are unsafe to use, may be provided with a needle guard 100 of the invention by simple manual assembly by e.g. a nurse or any other user prior to use. Hence, a needle guard 100 of the invention may recycle present unsafe medical equipment into safe medical equipment, once these needles cannot be used anymore due to safety legislations. Without such a versatile needle guard 100, such unsafe medical equipment would have to be discarded and replaced, to a high environmental and economic cost.

### The hinge structure 120

The dead-center toggle function of the hinge structure 120 is so arranged that it forces the needle shield 110 with a force to one of either of two oppositely-disposed positions, the open position or the closed position. In the closed position, the needle 210 is protected by the needle shield 110. In the open position, the needle 210 is exposed and may be used to penetrate the skin of a patient. When a greater opposite external force is applied, such as the force applied by a user, to the needle shield 110 or any extension thereof, in a direction from the open position and past the dead-center position, the hinge structure 120 snaps the needle shield 110 into the closed position, unless hindered by an external object such as e.g. the skin or clothing of a patient, just after it has passed the dead-center position.

Above described dead-center toggle function of the hinge structure 120 may be achieved by various assemblies, such as for example spring loaded hinges or other "snap" mechanisms, well known in the art.

The hinge structure 120 may comprise one or several toggle joints 121 and one or several tension members 124. A dead-center toggle function of the hinge structure 120 may thus be achieved. The one or several toggle joints 121 may be connected to spatially closely related portions of the needle shield 110 and the mounting base 140. Each toggle joint 121 may preferably consist of two relatively rigid leg portions 122 that are attached together at their ends in their longitudinal direction at a toggle joint knee 123. The toggle joint knee 123 may be flexible such that the leg portions 122 may be moved relative each other to allow movement of the needle shield 110 between closed and open position. The one or several tension members 124 may be able to resiliently stretch or expand to a limited amount. When stretched or expanded, a tension member 124 is elongated and strives to retain its original shorter length. Hence, a stretched or expanded tension member 124 will impose a force in the direction opposite the direction of the stretching or expansion. The point of attachment of a tension member 124 at the needle shield 110 is preferable above the point of attachment of the corresponding toggle joint 121. The point of attachment of a tension member 124 at the mounting base 140 is preferable below the point of attachment of the corresponding toggle joint 121. A tension member 124 may preferably be mounted close to the corresponding toggle joint 121, but preferably not so close that it may touch any part of the latter, whereby the intended function of the hinge structure 120 may otherwise be compromised. The dead-center position of the hinge structure 120 corresponds to specific angle, the dead-center angle, between the longitudinal extension of the needle 210 and the longitudinal extension of the needle shield 110. If the angle between the longitudinal extension of the needle 210 and the longitudinal extension of the needle shield 110 is less than the dead-center angle, the needle shield 110 strives toward the closed position. If this angle is greater than the dead-center angle, the needle shield 110 strives toward the open position. The dead-center angle may be in the range of 45 to 170°, such as 80 to 120° or 90 to 110°. The one skilled in the art may easily configure the hinge structure 120 to achieve a desired dead-center angle. For example, longer leg portions 122 will result in an increase of the dead-center angle and moving the point of attachment of a tension member 124 further above the point of attachment of the corresponding toggle joint 121 will result in a decrease of the dead-center angle.

According to one embodiment, the hinge structure 120 may comprise at least one, such as one, hinge and at least one, such as one, spring.

According to one embodiment, the hinge structure 120 may comprise one toggle joint 121 and one tension member 124, which may be placed in close proximity.

According to one embodiment, the hinge structure 120 may comprise two toggle joints 121 and one tension member 124, which may be placed in close proximity. The tension member 124 may be placed between the two toggle joints 121.

According to one embodiment, the hinge structure 120 may comprise one tension member 124 which is V-shaped and extending outwards so that it can be easily pressed on by a user. The distal end, i.e. the point of the V of the V-shaped tension member 124, may be shaped as or comprise a press member 125. The user may then advantageously activate the safety mechanism by pressing on the tension member 124 or the press member 125 thereof with, for example, a finger of the same hand which is holding the syringe. The press member 125 is configured, as known in the art, to be easily reached be the user and to be pressed on with a finger. Its outer surface, which the user presses on, may be planar or slightly convex or concave. It may be equipped with groves or elevations extending in a direction essentially perpendicular to the extension of the needle shield 110. Such groves or elevations minimize the risk of the finger slipping of the press member 125 when the user is pushing it.

### The locking mechanism

The locking mechanism of the needle guard 100 secures the needle shield 110 in closed position so that it cannot be moved back to an open position, or any other position where the needle 210 is exposed. Preferably, the locking mechanism is automatically activated when the needle shield 110 enters the closed position. The force, kinetic energy or momentum provided by the hinge structure 120 as it moves the needle shield 110 into the closed position may be sufficient to activate the locking mechanism. The locking mechanism may be, for example, a part of or an extension of the needle shield 110. It may also be a recess in the needle shield 110 that a corresponding protrusion connected to e.g. the mounting base 140 mates with in the closed position. When the locking mechanism is activated, the needle shield 110 is mechanically connected to, for example, the needle 110, the needle hub 220 or the mounting base 140, by the locking mechanism. The mounting base 140 may be provided with features or elements that enables or facilitate the locking of the locking mechanism, as known in the art. It may, for example be provided with a planar surface with an edge on which the locking member 132 slides and locks around, respectively, when moved into closed position. It may also be provided with e.g. a recess or similar, that a part of a locking member 132 mates with when locking.

According to one embodiment, the locking mechanism may comprise one or several, such as one or two, locking members 132 extending from or forming a part of the needle shield 110. A locking member 132 may be a hooked formed extension of the needle shield 110. It may be resiliently bended out of its resting state upon initial contact with the mounting base 140 in the movement towards the closed position. Upon entering the closed position, a part of the locking member 132, such as a distal hook-shaped end, may snap into a corresponding recess of the mounting base 140, or around an edge of the mounting base 140, and thereby lock the needle shield 110 in the closed position.

According to one embodiment, the locking mechanism may comprise one or several, such as one or two, locking members 132 provided with one or several slits 131. Such a slit 131 may be located at the side of the locking member 132 which is facing the mounting base 140 when the needle shield 110 is in closed position. The slit 131 may be a cut through a part, such as 10 to 95% or 25 to 75%, of the locking member 132 in a direction essentially perpendicular to its longitudinal extension. It may be located near the point of attachment of the locking members 132 to the needle shield 110, such as 0 to 60% or 0 to 40% of the total length of the longitudinal extension of the locking members 132 from the point of attachment. A slit 131 may also be a cut through a part of the needle shield 110 close to the attachment point of the a locking member 132. The depth or extension of such a slit 131 may be equal to or greater than the width of the locking member 132. Advantages of one or several slits 131 include, for example, the decrease in force needed for moving the needle shield 110 into the closed position. A locking member 132 is more easily bended out of its resting state upon initial contact with the mounting base 140 when provided with one or several slits 131. The risk of not achieving an automatic activation of the locking mechanism, by e.g. the force provided by the hinge structure 120, is thereby minimized.

According to one embodiment, the locking mechanism may comprise one or several, such as one or two, unidirectionally gated bars 130. Such bars may be extending from the inside and within the needle shield 110. Hence, such bars may be extending in the volume enclosed by the needle shield 110, for example, in a plane essentially perpendicular to the longitudinal extension of the needle shield 110. The longitudinal extension of a unidirectionally gated bar 130 may be in a direction from the elongated slot 115 towards the inside of the needle shield 110. The angle between the longitudinal extension of a unidirectionally gated bar 130 and the plane of the needles 210 path during movement from open to closed position may thus be, for example, 20 to 85°, such as 30 to 80° or 40 to 75°. The needle 210 thereby initially contacts a unidirectionally gated bar 130 at a point thereof on an inwards leaning slope thereof, when the needle shield is moved towards closed position. Preferably, a unidirectionally gated bar 130 is adapted such that it allows facile entry of the shaft of the needle 210, i.e. to close around the shaft of the needle 210, but prevents return of the needle 210 into any position where the needle 210 is not contained in the inner volume of the needle shield 110. The events that occur during activation of a locking mechanism, comprising at least one unidirectionally gated bar 130, when the needle shield 110 is moved towards an into the closed position, may be: (i) contact between the shaft of the needle 210 at a point on the surface of a unidirectionally gated bar 130 which is facing the elongated slot 115; (ii) the unidirectionally gated bar 130 is resiliently bending away from the force impressed by the shaft of the needle 210 so that the shaft slides inwards on the inwards leaning slope thereof; (iii) the shaft reaches the distal end of the unidirectionally gated bar 130, which resiliently returns to its initial configuration, i.e. its relaxed state, and traps the needle 210 in the needle shield 110. The distance between the distal end of a unidirectionally gated bar 130 in its relaxed state and the distal end of another oppositely placed unidirectionally gated bar 130 in its relaxed state, or the inner surface of the needle shield 110, is preferably less than the diameter of the shaft of the needle 210.

According to one embodiment, the locking mechanism may comprise one or several, such as one or two, unidirectionally gated bars 130 provided with one or several slits 131. Such a slit 131 may preferably be located at the side of the unidirectionally gated bar 130 facing the needle 210 when moved towards closed position. A slit 131 may be located anywhere along the extension of the unidirectionally gated bar 130 from its attachment point at the needle shield 110 to the point where the needle 210 is first contacting the unidirectionally gated bar 130 when moved into closed position. A slit 131 may thus be located on the bar such that the needle 210 never contacts it from its initial contact point with the bar, to the point where the bar traps the needle inside the needle shield 110. The slit 131 may be a cut through a part, such as 10 to 95% or 25 to 75%, of the unidirectionally gated bar 130 in a direction essentially perpendicular to its longitudinal extension. It may be located near the point of attachment of the unidirectionally gated bar 130 to the needle shield 110, such as 0 to 50% or 0 to 40% of the total length of the longitudinal extension of the unidirectionally gated bar 130 from the point of attachment. Advantages of one or several slits 131 include, for example, the decrease in force needed for moving the needle shield 110 into the closed position. A unidirectionally gated bar 130 is more easily bended out of its resting state upon initial contact with the needle 210 when provided with one or several slits 131. The risk of not achieving an automatic activation of the locking mechanism, by e.g. the force provided by the hinge structure 120, is thereby minimized.

According to one embodiment, the locking mechanism may comprise one or several, such as one or two, unidirectionally gated bars 130 extending from the same inner side of the needle shield 110 and towards the opposite inner side of the needle shield 110.

According to one embodiment, the locking mechanism may comprise one or several, such as one or two, pairs of unidirectionally gated bars 130. The unidirectionally gated bars 130 of each pair may be extending from the opposite inner sides of the needle shield 110 and towards each other. The distal ends may contact each other in the relaxed state, or the distance between these ends may preferably be less than the diameter of the needle 210, when in the relaxed state.

### Materials and manufacturing

The needle guard 100, or separate parts thereof, may be made of a suitable material such as, for example, a thermoplastic, plastic or polymeric material. Suitable materials for accomplishment of inherent resiliency in e.g. hinge structure 120, unidirectionally gated bar 130 or locking member 132 are well known in the art. Examples of such suitable materials include, but is not limited to, polypropylene (PP), high-density polyethylene (HDPE), polyamide (PA) and acrylnitrile-butadiene-styrene copolymer (ABS). Preferably, a material such as polypropylene (PP) is selected. These materials all allow for manufacturing of the needle guard 100 as a monolithically formed single article, which is highly beneficial with regard to keeping manufacturing costs down. Preferably, the thermoplastic, plastic or polymeric material has a suitable combination, for its intended purpose, of tenacity, rigidity, fatigue resistance, elasticity, and creep deformation resistance. The selection of a suitable thermoplastic, plastic or polymeric material may easily be made by the one skilled in the art. The one skilled in the art may also perform standard experiments in order to screen a range of thermoplastic, plastic or polymeric materials, whereby a suitable material may be selected on the basis of the results of such experiments. The manufacturing of needle guard 100 of the invention, or a needle guard assembly comprising needle guard 100, may comprise one or several steps selected from the group of steps consisting of: provision of a thermoplastic, plastic or polymeric material as starting material for needle guard 100; molding or injection molding a thermoplastic, plastic or polymeric material into one monolithically formed needle guard 100, which may or may not comprise the needle hub 220; and mounting needle guard 100 on needle hub 220 or the needle 210 in needle hub 220 by, for example, press-fitting or gluing mounting base 140 to needle hub 220 or the needle 210 to needle hub 220.

According to one embodiment, the needle guard 100 is made of a thermoplastic, plastic or polymeric material, as known in the art. An advantage of the use of a thermoplastic, plastic or polymeric material for the construction of the needle guard 100, in comparison to e.g. metal, is the greater freedom of variation of various details of the same. For example, a plastic needle guard 100 may be more conveniently molded than the corresponding metallic article. Another advantage includes the possibility to colour-code a plastic needle guard 100, for example according to the needle size. Yet another advantage of a plastic or polymeric needle guard 100 is the higher chemical inertness and/or resistance, in comparison to e.g. metal, towards e.g. corrosion. Yet another advantage of needle guard 100 of the invention, like a plastic or polymeric needle guard 100, is that it may be molded and produced in one functional piece, i.e. it does not have to be assembled by the combination of more than one separate article like other corresponding devices of the prior art. Hence, a reduction in the cost of production is resulting.

In the claims, the term "comprises/comprising" does not exclude the presence of other elements or steps. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A needle guard (100) for protecting a needle arranged in connection to said needle guard (100) from accidental contact, comprising:
a mounting base (140), having a lumen with a central axis, such that the needle arranged in connection to said needle guard (100) is extending substantially along said central axis;
a spring loaded needle shield (110), arranged at said mounting base (140), and being pivotable from an open position to a closed position, such that said spring loaded needle shield (110) may be pivoted from said open position, in which said needle shield (110) is separated from said needle, to said closed position, in which said needle shield (110) is enclosing and protecting said needle;
a hinge structure (120) connecting said needle shield (110) to said mounting base (140) for forcing said needle shield (110) into one of either said open position or said closed position, wherein said hinge structure (120) is comprising at least one toggle joint (121) and at least one tension member (124), said at least one toggle joint (121) and at least one tension member (124) being connected to said needle shield (110) and to said mounting base (140);
said mounting base (140) being movably connected to said needle shield (110) for enabling movement between said open and said closed position;
wherein said needle shield (110) is having a passive state and an active state, said needle shield (110) being urged towards said closed position when in said active state;
wherein said hinge structure (120) has a dead-center position, such that when said needle shield (110) is in said passive state the needle shield (110) is being forced by said hinge structure (120) into said open position if said dead-center position has to be passed to reach said closed position, and when the needle shield (110) is in said active state the needle shield (110) is being forced by said hinge structure (120) into said closed position if said dead-center position has to be passed to reach said open position; and
**characterized in that**
said dead-center position is a position in which the angle between the central axis lumen of the mounting base (140) and the longitudinal extension of said needle shield (110) is in the range of 45 to 170°;
wherein, after the user activates the safety function by manually moving the needle shield (110) passed the dead-center position, the needle guard (100) automatically, without the need of any further additional act by the user, protects the needle (210) immediately after withdrawal of the needle from the skin of a patient.

2. The needle guard (100) according to claim 1, wherein an injection needle (210) is arranged at the distal end of the mounting base, said needle (210) extending from said mounting base along said central axis, wherein a lumen of the injection needle (210) communicates with the lumen of the mounting base (140), such that the a spring loaded needle shield (110) may be pivoted from said open position, in which said needle shield (110) is separated from said injection needle (210), to a closed position, in which said needle shield (110) is enclosing and protecting said injection needle (210);
said mounting base (140) having a constant spatial relationship with said injection needle (210) when said needle guard (100) is being used.

3. The needle guard according to claim 1, wherein said mounting base (140) comprises means for connection to both a needle hub (220), from which needle hub (220) an injection needle (210) is extending, and a syringe (310).

4. The needle guard according to claim 3, wherein said means for connection comprise at least one of: a male fitting (150), for connection to said needle hub (220); and a female fitting (151), for connection to said syringe (310).

5. The needle guard (100) according to any one of the preceding claims, wherein said mounting base (140) comprises a needle hub bore (141) for attachment to a needle hub (220), from which needle hub (220) said needle (210) is extending.

6. The needle guard (100) according to any one of claims 1 or 2, wherein said mounting base (140) is integrated with said needle hub (220), from which needle hub (220) said needle (210) is extending, such that said mounting base (140) and said needle hub (220) are parts of the same monolithically formed article.

7. The needle guard (100) according any one of the preceding claims, further comprising at least one locking member (132) extending from or forming a part of said needle shield (110), wherein said at least one locking member (132) is lockingly engaging with said mounting base (140) or any extension or recess thereof when said needle shield (110) is in said closed position, to lock said needle shield (110) in said closed position.

8. The needle guard (100) according to any one of the preceding claims, wherein said locking member (132) is comprising at least one slit (131), for allowing said needle shield (110) to lockingly engage with said mounting base (140) by utilization of a relatively low force.

9. The needle guard (100) according to any of the preceding claims, further comprising at least one unidirectionally gated bar (130) extending from said needle shield (110) in the volume enclosed by said needle shield (110), said unidirectionally gated bar (130) lockingly engaging with said needle (210) when said needle shield (110) is in said closed position, to lock said needle shield (110) in said closed position.

10. The needle guard (100) according to claim 9, wherein said unidirectionally gated bar (130) is comprising at least one slit (131), for allowing said needle shield (110) to lockingly engage with said needle (210) by utilization of a relatively low force.

11. The needle guard (100) according to any one of claims 7 to 10, wherein the force needed for said needle shield (110) to lockingly engage with said mounting base (140) or said needle (210) is less that the force by which said needle shield (110) is being urged towards said closed position when in said active state, for allowing locking of said needle shield (110) in said closed position by the force by which said needle shield (110) is being urged towards said closed position when in said active state.

12. The needle guard (100) according to any one of the preceding claims, wherein the needle guard (100) is monolithically formed as a single article when manufactured.

13. The needle guard (100) according to any one of the preceding claims, being made of a thermoplastic, plastic or polymeric material.

14. A needle guard assembly, comprising a needle guard (100) according to any one of claims 1 to 13, a needle hub (220) and a needle (210), wherein said needle guard (100) is mounted on, connected to or integrated with said needle hub (220).

## Patentansprüche

1. Nadelschutz (100) zum Schutz einer in Verbindung mit dem Nadelschutz (100) angeordneten Nadel vor unbeabsichtigtem Kontakt, umfassend:
eine Befestigungsbasis (140) mit einem Lumen mit einer Mittelachse, so dass sich die Nadel, die in Verbindung mit dem Nadelschutz (100) angeordnet ist, im Wesentlichen entlang der Mittelachse erstreckt,
eine federbelastete Nadelabschirmung (110), die an der Befestigungsbasis (140) angeordnet ist und von einer offenen Position in eine geschlossene Position verschwenkbar ist, so dass die federbelastete Nadelabschirmung (110) von der offenen Position, in der die Nadelabschirmung (110) von der Nadel getrennt ist, in die geschlossene Position geschwenkt werden kann, in der die Nadelabschirmung (110) die Nadel umschließt und schützt;
eine Gelenkstruktur (120), die die Nadelabschirmung (110) mit der Befestigungsbasis (140) verbindet, um die Nadelabschirmung (110) entweder in die offene Position oder die geschlossene Position zu zwingen, wobei die Gelenkstruktur (120) mindestens ein Kippgelenk (121) und mindestens ein Spannelement (124) umfasst, wobei das zumindest eine Kippgelenk (121) und das zumindest eine Spannelement (124) mit der Nadelabschirmung (110) und der Befestigungsbasis (140) verbunden sind;
die Befestigungsbasis (140) beweglich mit der Nadelabschirmung (110) verbunden ist, um eine Bewegung zwischen der offenen und der geschlossenen Position zu ermöglichen,
wobei die Nadelabschirmung (110) einen passiven Zustand und einen aktiven Zustand aufweist, und die Nadelabschirmung (110) in Richtung der geschlossenen Position gedrängt wird, wenn sie sich im aktiven Zustand befindet;
wobei die Gelenkstruktur (120) eine Totpunktposition aufweist, so dass, wenn sich die Nadelabschirmung (110) im passiven Zustand befindet, die Nadelabschirmung (110) durch die Gelenkstruktur (120) in die offene Position gedrängt wird, wenn die Totpunktposition passiert werden muss, um die geschlossene Position zu erreichen, und
wenn sich die Nadelabschirmung (110) im aktiven Zustand befindet, die Nadelabschirmung (110) durch die Gelenkstruktur (120) in die geschlossene Position gedrängt wird, wenn die Totpunktposition passiert werden muss, um die offene Position zu erreichen; und
**dadurch gekennzeichnet, dass** die Totpunktposition eine Position ist, in der der Winkel zwischen dem Mittelachsenlumen der Befestigungsbasis (140) und der Längserstreckung der Nadelabschirmung (110) im Bereich von 45 bis 170° liegt;
wobei, nachdem der Benutzer die Sicherheitsfunktion durch manuelles Bewegen der Nadelabschirmung (110) über die Totpunktposition hinaus aktiviert hat, der Nadelschutz (100) die Nadel (210) unmittelbar nach dem Zurückziehen der Nadel aus der Haut eines Patienten automatisch schützt, ohne dass der Benutzer eine weitere zusätzliche Handlung ausführen muss.

2. Nadelschutz (100) nach Anspruch 1, wobei eine Injektionsnadel (210) am distalen Ende der Befestigungsbasis angeordnet ist, und sich die Nadel (210) von der Befestigungsbasis entlang der Mittelachse erstreckt, wobei ein Lumen der Injektionsnadel (210) mit dem Lumen der Befestigungsbasis (140) kommuniziert, so dass die federbelastete Nadelabschirmung (110) von der offenen Position, in der die Nadelabschirmung (110) von der Injektionsnadel (210) getrennt ist, in eine geschlossene Position verschwenkt werden kann, in der die Nadelabschirmung (110) die Injektionsnadel (210) umschließt und schützt;
und die Befestigungsbasis (140) eine konstante räumliche Beziehung zur Injektionsnadel (210) aufweist, wenn der Nadelschutz (100) in Gebrauch ist.

3. Nadelschutz (100) nach Anspruch 1, wobei die Befestigungsbasis (140) Mittel zur Verbindung sowohl mit einem Nadelansatz (220), von welchem Nadelansatz (220) sich eine Injektionsnadel (210) erstreckt, als auch mit einer Spritze (310) umfasst.

4. Nadelschutz (100) nach Anspruch 3, wobei die Mittel zur Verbindung mindestens eines der folgenden umfassen: ein männliches Anschlussstück (150) zur Verbindung mit dem Nadelansatz (220); und ein weibliches Anschlussstück (151) zur Verbindung mit der Spritze (310).

5. Nadelschutz (100) nach einem der vorhergehenden Ansprüche, wobei die Befestigungsbasis (140) eine Nadelansatzbohrung (141) zur Befestigung an einem Nadelansatz (220) umfasst, von welchem Nadelansatz (220) sich die Nadel (210) erstreckt.

6. Nadelschutz (100) nach einem der Ansprüche 1 oder 2, wobei die Befestigungsbasis (140) in den Nadelansatz (220) integriert ist, von welchem Nadelansatz (220) sich die Nadel (210) erstreckt, so dass die Befestigungsbasis (140) und der Nadelansatz (220) Teile desselben monolithisch geformten Gegenstands sind.

7. Nadelschutz (100) nach einem der vorhergehenden Ansprüche, der ferner zumindest ein Verriegelungselement (132) umfasst, das sich von der Nadelabschirmung (110) erstreckt oder einen Teil davon bildet, wobei das zumindest eine Verriegelungselement (132) in verriegelndem Eingriff mit der Befestigungsbasis (140) oder einer Verlängerung oder Aussparung davon steht, wenn sich die Nadelabschirmung (110) in der geschlossenen Position befindet, um die Nadelabschirmung (110) in der geschlossenen Position zu verriegeln.

8. Nadelschutz (100) nach einem der vorhergehenden Ansprüche, wobei das Verriegelungselement (132) zumindest einen Schlitz (131) umfasst, damit die Nadelabschirmung (110) unter Verwendung einer relativ geringen Kraft verriegelnd mit der Befestigungsbasis (140) in Eingriff gebracht werden kann.

9. Nadelschutz (100) nach einem der vorstehenden Ansprüche, der ferner zumindest einen in einer Richtung begrenzten Steg (130) umfasst, der sich von der Nadelabschirmung (110) in das von der Nadelabschirmung (110) umschlossene Volumen erstreckt, welcher in einer Richtung begrenzte Steg (130) mit der Nadel (210) verriegelt in Eingriff steht, wenn sich die Nadelabschirmung (110) in der geschlossenen Position befindet, um die Nadelabschirmung (110) in der geschlossenen Position zu verriegeln.

10. Der Nadelschutz (100) nach Anspruch 9, wobei der in einer Richtung begrenzte Steg (130) zumindest einen Schlitz (131) umfasst, damit die Nadelabschirmung (110) unter Verwendung einer relativ geringen Kraft verriegelnd mit der Nadel (210) in Eingriff gebracht werden kann.

11. Nadelschutz (100) nach einem der Ansprüche 7 bis 10, wobei die Kraft, die erforderlich ist, damit die Nadelabschirmung (110) verriegelnd mit der Befestigungsbasis (140) oder der Nadel (210) in Eingriff kommt, geringer ist als die Kraft, durch die die Nadelabschirmung (110) in Richtung der geschlossenen Position gedrängt wird, wenn sie sich im aktiven Zustand befindet, um ein Verriegeln der Nadelabschirmung (110) in der geschlossenen Position durch die Kraft zu ermöglichen, durch die die Nadelabschirmung (110) in Richtung der geschlossenen Position gedrängt wird, wenn sie sich im aktiven Zustand befindet.

12. Nadelschutz (100) nach einem der vorhergehenden Ansprüche, wobei der Nadelschutz (100) bei der Herstellung monolithisch als ein einziger Gegenstand geformt ist.

13. Nadelschutz (100) nach einem der vorhergehenden Ansprüche, wobei er aus einem thermoplastischen, plastischen oder polymeren Material besteht.

14. Nadelschutzanordnung, umfassend einen Nadelschutz (100) nach einem der Ansprüche 1 bis 13, einen Nadelansatz (220) und eine Nadel (210), wobei der Nadelschutz (100) an dem Nadelansatz (220) angebracht, mit diesem verbunden oder in diesen integriert ist.

## Revendications

1. Protection d'aiguille (100) pour protéger une aiguille agencée en connexion avec ladite protection d'aiguille (100) à l'encontre un contact accidentel, comprenant :
une base de montage (140), présentant une lumière avec un axe central, de telle sorte que l'aiguille agencée en connexion avec ladite protection d'aiguille (100) s'étende sensiblement le long dudit axe central ;
une gaine d'aiguille chargée par ressort (110), agencée au niveau de ladite base de montage (140), et pouvant pivoter d'une position ouverte à une position fermée, de sorte que ladite gaine d'aiguille chargée par ressort (110) peut être pivotée de ladite position ouverte, dans laquelle ladite gaine d'aiguille (110) est séparée de ladite aiguille, à ladite position fermée, dans laquelle ladite gaine d'aiguille (110) renferme et protège ladite aiguille ;
une structure articulée (120) connectant ladite gaine d'aiguille (110) à ladite base de montage (140) pour forcer ladite gaine d'aiguille (110) dans une de ladite position ouverte ou de ladite position fermée, dans laquelle ladite structure articulée (120) comprend au moins une genouillère (121) et au moins un élément de tension (124), ladite au moins une genouillère (121) et ledit au moins un élément de tension (124) étant connectés à ladite gaine d'aiguille (110) et à ladite base de montage (140) ;
ladite base de montage (140) étant connectée de manière mobile à ladite gaine d'aiguille (110) pour permettre un déplacement entre ladite position ouverte et ladite position fermée ;
dans laquelle ladite gaine d'aiguille (110) présente un état passif et un état actif, ladite gaine d'aiguille (110) étant sollicitée vers ladite position fermée lorsqu'elle est dans ledit état actif ;
dans laquelle ladite structure articulée (120) présente une position de point mort, de sorte que lorsque ladite gaine d'aiguille (110) est dans ledit état passif, la gaine d'aiguille (110) est forcée par ladite structure articulée (120) dans ladite position ouverte si ladite position de point mort doit être dépassée pour atteindre ladite position fermée, et lorsque la gaine d'aiguille (110) est dans ledit état actif, la gaine d'aiguille (110) est forcée par ladite structure articulée (120) dans ladite position fermée si ladite position de point mort doit être dépassée pour atteindre ladite position ouverte ; et
**caractérisée en ce que** ladite position de point mort est une position dans laquelle l'angle entre la lumière d'axe central de la base de montage (140) et l'extension longitudinale de ladite gaine d'aiguille (110) est dans la plage de 45 à 170° ;
dans laquelle, après que l'utilisateur a activé la fonction de sécurité en déplaçant manuellement la gaine d'aiguille (110) au-delà de la position de point mort, la protection d'aiguille (100) protège automatiquement l'aiguille (210) immédiatement après le retrait de l'aiguille à partir de la peau d'un patient, sans avoir besoin d'un quelconque autre acte supplémentaire de la part de l'utilisateur.

2. Protection d'aiguille (100) selon la revendication 1, dans laquelle une aiguille d'injection (210) est agencée à l'extrémité distale de la base de montage, ladite aiguille (210) s'étendant à partir de ladite base de montage le long dudit axe central, dans laquelle une lumière de l'aiguille d'injection (210) communique avec la lumière de la base de montage (140), de sorte que la gaine d'aiguille chargée par ressort (110) peut être pivotée de ladite position ouverte, dans laquelle ladite gaine d'aiguille (110) est séparée de ladite aiguille d'injection (210), à une position fermée, dans laquelle ladite gaine d'aiguille (110) renferme et protège ladite aiguille d'injection (210) ;
ladite base de montage (140) présentant une relation spatiale constante avec ladite aiguille d'injection (210) lorsque ladite protection d'aiguille (100) est utilisée.

3. Protection d'aiguille selon la revendication 1, dans laquelle ladite base de montage (140) comprend des moyens pour une connexion à la fois à un moyeu d'aiguille (220), moyeu d'aiguille (220) à partir duquel une aiguille d'injection (210) s'étend, et à une seringue (310).

4. Protection d'aiguille selon la revendication 3, dans laquelle lesdits moyens de connexion comprennent au moins un parmi : un raccord mâle (150), pour connexion audit moyeu d'aiguille (220) ; et un raccord femelle (151), pour connexion à ladite seringue (310).

5. Protection d'aiguille (100) selon l'une quelconque des revendications précédentes, dans laquelle ladite base de montage (140) comprend un alésage de moyeu d'aiguille (141) pour fixation à un moyeu d'aiguille (220), moyeu d'aiguille (220) à partir duquel ladite aiguille (210) s'étend.

6. Protection d'aiguille (100) selon l'une quelconque des revendications 1 ou 2, dans laquelle ladite base de montage (140) est intégrée audit moyeu d'aiguille (220), moyeu d'aiguille (220) à partir duquel ladite aiguille (210) s'étend, de sorte que ladite base de montage (140) et ledit moyeu d'aiguille (220) sont des parties du même article formé de manière monolithique.

7. Protection d'aiguille (100) selon l'une quelconque des revendications précédentes, comprenant en outre au moins un élément de verrouillage (132) s'étendant à partir de ou formant une partie de ladite gaine d'aiguille (110), dans laquelle ledit au moins un élément de verrouillage (132) est mis en prise de verrouillage avec ladite base de montage (140) ou toute extension ou évidement de celle-ci lorsque ladite gaine d'aiguille (110) est dans ladite position fermée, pour verrouiller ladite gaine d'aiguille (110) dans ladite position fermée.

8. Protection d'aiguille (100) selon l'une quelconque des revendications précédentes, dans laquelle ledit élément de verrouillage (132) comprend au moins une fente (131), pour permettre à ladite gaine d'aiguille (110) de venir en prise de verrouillage avec ladite base de montage (140) en utilisant une force relativement faible.

9. Protection d'aiguille (100) selon l'une quelconque des revendications précédentes, comprenant en outre au moins une barre à porte unidirectionnelle (130) s'étendant à partir de ladite gaine d'aiguille (110) dans le volume renfermé par ladite gaine d'aiguille (110), ladite barre à porte unidirectionnelle (130) venant en prise de verrouillage avec ladite aiguille (210) lorsque ladite gaine d'aiguille (110) est dans ladite position fermée, pour verrouiller ladite gaine d'aiguille (110) dans ladite position fermée.

10. Protection d'aiguille (100) selon la revendication 9, dans laquelle ladite barre à porte unidirectionnelle (130) comprend au moins une fente (131), pour permettre à ladite gaine d'aiguille (110) de venir en prise de verrouillage avec ladite aiguille (210) en utilisant une force relativement faible.

11. Protection d'aiguille (100) selon l'une quelconque des revendications 7 à 10, dans laquelle la force nécessaire pour que ladite gaine d'aiguille (110) vienne en prise de verrouillage avec ladite base de montage (140) ou ladite aiguille (210) est inférieure à la force par laquelle ladite gaine d'aiguille (110) est sollicitée vers ladite position fermée lorsqu'elle est dans ledit état actif, pour permettre le verrouillage de ladite gaine d'aiguille (110) dans ladite position fermée par la force par laquelle ladite gaine d'aiguille (110) est sollicitée vers ladite position fermée lorsqu'elle est dans ledit état actif.

12. Protection d'aiguille (100) selon l'une quelconque des revendications précédentes, dans laquelle l'élément de protection d'aiguille (100) est formé de manière monolithique en tant sous la forme d'un article unique lors de sa fabrication.

13. Protection d'aiguille (100) selon l'une quelconque des revendications précédentes, constituée d'un matériau thermoplastique, plastique ou polymère.

14. Ensemble de protection d'aiguille, comprenant une protection d'aiguille (100) selon l'une quelconque des revendications 1 à 13, un moyeu d'aiguille (220) et une aiguille (210), dans lequel ladite protection d'aiguille (100) est montée sur, connectée à ou intégrée audit moyeu d'aiguille (220).
